Europäisches Patentamt

European Patent Office

Office européen des brevets

(19)

(11) Publication number: **0 245 105**
A1

# EUROPEAN PATENT APPLICATION

(21) Application number: **87304103.2**

(22) Date of filing: **08.05.87**

(51) Int. Cl.⁴: **A 61 K 33/00,** A 61 K 33/14
// (A61K33/00, 31:70),(A61K33/14, 33:00, 31:70, 31:19)

(30) Priority: **09.05.86 GB 8611343**
**13.12.86 GB 8629831**

(43) Date of publication of application: **11.11.87**
**Bulletin 87/46**

(84) Designated Contracting States: **ES GR**

(71) Applicant: **BEECHAM GROUP PLC, Beecham House
Great West Road, Brentford Middlesex TW8 9BD (GB)**

(72) Inventor: **Bywater, Robert James, Beecham
Pharmaceuticals Walton Oaks Dorking Road, Tadworth
Surrey KT20 7NT (GB)**
Inventor: **Dupe, Robert John, Beecham Pharmaceuticals
Walton Oaks Dorking Road, Tadworth Surrey KT20 7NT
(GB)**
Inventor: **Wylie, David Ferguson, Beecham
Pharmaceuticals Walton Oaks Dorking Road, Tadworth
Surrey KT20 7NT (GB)**

(74) Representative: **West, Vivien et al, Beecham
Pharmaceuticals Great Burgh Yew Tree Bottom Road,
Epsom Surrey KT18 5XQ (GB)**

(54) Veterinary composition.

(57) An aqueous veterinary composition comprises: not less than 300 parts sodium ion; 10 to 15 parts potassium ion; not less than 95 parts chloride ion; 200 to 400 parts bicarbonate ion and/or one or more metabolic precursors thereof such as acetate, lactate or citrate; 0.1 to 1.0 parts magnesium ion, 5 to 15 (additional) parts citrate ion, and not more than 1000 parts dextrose, the composition having a total osmolarity of 1100 to 2400 mOsmoll⁻¹, preferably about 1600 mOsmoll⁻¹, and is useful in the treatment of shock by intravenous infusion.

EP 0 245 105 A1

## VETERINARY COMPOSITION

This invention relates to veterinary compositions useful for treating disorders in domestic animals, and in particular to compositions useful for treating shock in domestic animals and to the use of such compositions in the treatment of shock by intravenous infusion.

Common causes of shock in domestic animals include coli mastitis in cows (which is an example of endotoxin shock), acute dehydration, such as in scouring calves, and blood loss in dogs and cats such as follows accident or injury. The symptoms typically include hypovolaemia, reduced cardiac output and peripheral perfusion, and acidosis. Hypoglycaemia and hyperkalaemia may also occur.

Previously proposed methods of treatment nave therefore included rehydration using a large volume of liquid which is administered orally or parenterally.

GB-A-1,581,826 describes an oral rehydration composition for use in the treatment of scours, post-operative dehydration and stress, as well as a method of treatment which comprises administering an aqueous solution of the composition. The composition comprises 40 to 80% by weight of an actively absorbed monosaccharide such as glucose, 7.5 to 30% by weight of an actively absorbed naturally occurring amino acid such as glycine, and 0.5 to 10% by weight of citric acid or a salt thereof such as tri-potassium citrate, as well as, optionally, sodium chloride and/or other ions such as bicarbonate.

0245105

- 2 -

FR-A-2,467,599 describes a rehydration composition for use in the treatment of scours which comprises an amino acid or a salt thereof such as sodium glutamate, a sodium carboxylate such as sodium acetate, and glucose, as well as, optionally, potassium, magnesium and/or bicarbonate ions. The composition is administered orally or intravenously as an approximately isotonic aqueous solution (isotonic = 300mOsmol $1^{-1}$).

The infusion of large volumes of isotonic saline solution in cases of shock also forms part of the state of the art.

A disadvantage of such prior art compositions has been the large volumes of liquid required and the long time period over which intravenous infusion thereof must be carried out.

It has been disclosed by Nakayama et al (''Circulatory Shock'' 13: 149-159 (1984)) that the injection of small volumes (3-4ml/kg in shocked adult female sheep) of hypertonic (2,400mOsmoll$^{-1}$) saline solution could partially restore plasma volume, cardiac output and peripheral perfusion to a significant extent in the short term.

New compositions have now been discovered which combine effectiveness in the treatment of shock in domestic animals with ease of formulation and useful stability.

Accordingly, the present invention provides an aqueous veterinary composition comprising: not less than 300 parts sodium ion; 10 to 15 parts potassium ion; not less than 95 parts chloride ion; 200 to 400 parts bicarbonate ion and/or one or more metabolic precursors thereof such as acetate, lactate or citrate; 0.1 to 1.0 parts magnesium ion, 5 to 15 (additional) parts citrate ion, and not more than 1000 parts dextrose, the composition having a total osmolarity of 1100 to 2400 mOsmol l$^{-1}$, preferably about 1600 mOsmol l$^{-1}$. All quantities specified herein are calculated on the basis of the number of mole equivalents used.

Advantageously, to enhance stability on storage, the dextrose and the other solute components of the composition are stored separately in two containers as respective aqueous solutions which are mixed together immediately prior to use. Typically the two solutions will be of approximately equal osmolarity. Thus, in one embodiment of the invention the dextrose solution will contain 500 to 1200 mmoles dextrose per 500 ml of water and the other solution to be admixed therewith will contain 610 to 1200 mmoles ions per 500 ml of water. Typically, the two solutions will be mixed together, for example in approximately equal quantities, to obtain an aqueous veterinary composition according to the invention.

The two containers will typically be made of a plastics material suitable for large volume intravenous infusions, such as high or low density polyethylene or plasticised PVC. The containers may be rigid, semi-rigid or collapsible and may be joined together in a suitable manner. In one embodiment, each container has two openings. One opening allows liquid filling and is sealable. The other opening has a plastics/elastomeric septum closure allowing a needle to pass through, thereby enabling the contents to be withdrawn. The composition will typically be administered by using a Y-piece giving set wherein the arms of the catheter have needles to empty the containers as described. The two streams of liquid

- 4 -

thus mix in the giving set before reaching the vein during administration.

The invention further provides a method of treating shock in domestic animals, which method comprises administering to the animal suffering from shock a said aqueous composition intravenously.

The invention additionally provides a process for preparing the said aqueous composition, which process comprises mixing together the ingredients in the necessary proportions.

The aqueous composition of the invention will typically be prepared by mixing together a dry powder composition and water.

Thus, the invention still further provides a dry powder composition comprising: not less than 300 parts sodium ion; 10 to 15 parts potassium ion; not less than 95 parts chloride ion; 200 to 400 parts bicarbonate ion and/or one or more metabolic precursors thereof such as acetate, lactate, or citrate; 0.1 to 1.0 parts magnesium ion, 5 to 15 (additional) parts citrate ion, and not more than 1000 parts dextrose.

According to one embodiment of the invention, the veterinary compositions will contain 300 to 700 parts sodium ion, 95 to 270 parts chloride ion, and 500 to 1000 parts dextrose.

The veterinary compositions of the invention will typically contain at least 80, more particularly 80 to 260, parts, for example 172 parts, sodium chloride.

- 5 -

The potassium ion may be presented as potassium bicarbonate or mono-, di or tripotassium citrate but is advantageously presented as 10 to 15 parts, more particularly about 13 parts, potassium chloride.

The citrate is preferably a sodium citrate such as trisodium citrate which may be anhydrous.

In one embodiment the veterinary composition of the invention will additionally include up to 5 parts calcium ion; more particularly about 3 parts calcium ion, which may be presented as the chloride.

If desired the compositions of this invention can contain other substances such as vitamins, minerals, amino acids, excipients or the like in conventional manner. Advantageously the aqueous composition of the invention may include preservative agents such as bacteriocides and/or fungicides to optimize storage life.

It will be realised that anti-bacterial agents may be administered in conjunction with the compositions of the invention. Examples of suitable anti-bacterial agents for such use include ampicillin, amoxycillin and tetracyclines.

In general the dry powder compositions of the invention may be prepared by mixing together the individual components in conventional manner. Advantageously, one or more of the components may be provided in anhydrous form, in order to optimize the stability of the dry powder composition. Once mixed the composition may be put into sachets or other conventional containers. The aqueous compositions of the invention may be prepared by dissolving the dry powder compositions of

- 6 -

the invention in water which preferably has been sterilized and made pyrogen-free.

Thus the compositions of the invention may be provided as a dry powder which can be dissolved in water as required, as an aqueous solution which is ready for use, in the form of a twin-pack in which the dry powder is provided separately from a container of sterile water, or in the form of a twin-pack in which a dextrose solution is provided separately from a solution of the other components of the composition.

In a particularly advantageous embodiment of the invention, an aqueous solution for intravenous injection comprises at least 300, more particularly 300 to 700, mmoles sodium ion, 10 to 15 mmoles potassium ion, at least 95, more particularly 95 to 270, mmoles chloride ion, 200 to 400 mmoles bicarbonate ion and/or the precursor(s) therefor, 0.1 to 1.0 mmoles magnesium ion, 5 to 15 mmoles citrate ion and not more than 1000, more particularly 500 to 1000, mmoles dextrose per litre of water. The osmolarity of such a solution is thus from 1110 to 2400 mOsmol l$^{-1}$.

A particular advantage of the aqueous composition of the invention is that only a small volume (3 to 15 ml/kg, for example, about 0.5l for a 50 kg calf and about 2.5l for a 500 kg cow) need be administered, and it can be administered over a short time period. This may be followed up as required with a further dose of the aqueous composition of the invention, or with conventional fluid therapy such as the administration of isotonic saline solution. The method of the invention is particularly suitable for use in the case when the animal is initially too weak to receive fluids via the oral route. The method of the invention

- 7 -

effects a rapid and effective reversal of shock, witn substantial restoration of cardiac output, peripheral perfusion and plasma volume and amelioration of hypoglycaemia and acidosis.

The following examples illustrate the invention:

- 8 -

Example 1

A total of 167.45g of the following composition was prepared by mixing together the following ingredients in dry powder form.

|                      | Grams | m Moles |
|----------------------|-------|---------|
| Sodium chloride      | 10    | 172     |
| Sodium bicarbonate   | 21    | 250     |
| Potassium chloride   | 1     | 13.4    |
| Anhydrous dextrose   | 132   | 733     |
| Magnesium chloride   | 0.05  | 0.5     |
| Trisodium citrate dihydrate | 3.4 | 12  |

The whole composition was then dissolved in 1 litre of water. Ionic analysis showed the ionic content of the solution to be as follows:

| Total ionic content | $mEq\,L^{-1}$ |
|---------------------|------|
| $Na^+$              | 458  |
| $K^+$               | 13   |
| $Cl^-$              | 185  |
| $HCO_3-$            | 250  |
| $Mg^{++}$           | Trace |
| citrate             | 12   |

The osmolarity of the solution was thus about 1,651 $mOsmo\,l^{-1}$.

Example 2

The effect of intravenous infusion of the aqueous solution of Example 1 was evaluated in acutely dehydrated calves. The method used was to anaesthetise calves, and then place cannulas for measurement of blood pressure and central venous pressure, and volume of voided urine. The calves were then made dehydrated by intravenous infusion of diuretic followed, after a period of diuresis, by intraperitoneal instillation of hypertonic mannitol solution. 4 to 5% dehydration was rapidly achieved before treatment with the aqueous solution of Example 1. 500ml of the solution were given by rapid intravenous infusion over about 8 minutes, and the animals were monitored for blood pressure, central venous pressure, volume of voided urine, heart rate/ECG and blood pH. Limb perfusion, blood pressure and central venous pressure were all improved, plasma volume was increased, and a low blood pH was either stabilized or improved in each subject. Limited control comparisons with 1500ml intravenous isotonic saline solution showed little improvement compared with 500ml volume of the solution of the invention. The same volume of hypertonic (1600 $mOsmoll^{-1}$) saline solution failed to improve the blood acid-base balance and caused high blood sodium levels. The same volume of intravenous 40% (40g/100l) dextrose solution was found to stimulate limb perfusion, but reduced plasma volume and impaired cardiac function. Plasma volume was measured using the Evans blue dye dilution technique (Gibson and Evans J. Clin. Invest [1937] 16, 301-316).

Claims

1.     An aqueous veterinary composition comprising: not less than 300 parts sodium ion; 10 to 15 parts potassium ion; not less than 95 parts chloride ion; 200 to 400 parts bicarbonate ion and/or one or more metabolic precursors thereof; 0.1 to 1.0 parts magnesium ion, 5 to 15 (additional) parts citrate ion, and not more than 1000 parts dextrose, the composition having a total osmolarity of 1100 to 2400 mOsmoll$^{-1}$.

2.     A composition according to claim 1, having an osmolarity of about 1600 mOsmoll$^{-1}$.

3.     A composition according to claim 1, wherein the metabolic precursor(s) of bicarbonate ion, if present, comprise acetate, lactate and/or citrate.

4.     A composition according to claim 1, comprising 300 to 700 parts sodium ion, 95 to 270 parts chloride ion, and 500 to 1000 parts dextrose.

5.     A composition according to claim 1, comprising a first aqueous solution containing dextrose and a second aqueous solution containing the other solute components of the composition, such that the first and second solutions can be admixed in predetermined proportions to obtain a solution having an osmolarity of 1100 to 2400 mOsmoll$^{-1}$.

6.     A dry powder composition comprising: not less than 300 parts sodium ion; 10 to 15 parts potassium ion; not less than 95 parts chloride ion; 200 to 400 parts bicarbonate ion and/or one or more metabolic precursors thereof; 0.1 to 1.0 parts magnesium ion, 5

- 11 -

to 15 (additional) parts citrate ion, and not more than 1000 parts dextrose.

7.    A process for the preparation of a composition according to any preceding claim, which process comprises mixing together the ingredients thereof in the necessary proportions.

8.    A method of treating shock in non-human animals, which method comprises administering to the animal suffering from shock an aqueous composition according to any one of claims 1 to 4 intravenously.

9.    A composition according to any one of claims 1 to 6, for use in the treatment of shock in non-human animals by intravenous infusion.

10.    Use of a composition according to any one of claims 1 to 6 for the manufacture of a medicament for use in the treatment of shock in non-human animals by intravenous infusion.

European Patent
Office

**PARTIAL EUROPEAN SEARCH REPORT**
which under Rule 45 of the European Patent Convention
shall be considered, for the purposes of subsequent
proceedings, as the European search report

Application number

**EP 87 30 4103**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | WO - A - 85 01657 (THE STATE OF VICTORIA) <br><br> * Page 14, line 1 - page 15, line 33; claims 1-8 * | 1-7, 9-10 | A 61 K 33/00 <br> A 61 K 33/14// <br> (A 61 K 33/00, <br> 31:70) <br> (A 61 K 33/14, <br> 33:00, 31:70, <br> 31:19) |
| A | UNLISTED DRUGS, vol. 34, no. 2, February 1982 Chatham, N.J., US <br><br> * Page 27 'Saloral' | 1-7, 9-10 | |

TECHNICAL FIELDS
SEARCHED (Int. Cl.4)

A 61 K

### INCOMPLETE SEARCH

The Search Division considers that the present European patent application does not comply with
the provisions of the European Patent Convention to such an extent that it is not possible to carry
out a meaningful search into the state of the art on the basis of some of the claims.
Claims searched completely:    1-7, 9-10
Claims searched incompletely:
Claims not searched:        8
Reason for the limitation of the search:

Method for treatment of the human or animal
body by surgery or therapy (see art. 52(4)
of the European Patent Convention).

| Place of search | Date of completion of the search | Examiner |
|---|---|---|
| THE HAGUE | 30-07-1987 | BRINKMANN |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO Form 1505.1. 03.82